# FASCICULE DE BREVET EUROPEEN

(11) **EP 0 627 212 B1**
(45) Date de publication et mention de la délivrance du brevet: **16.04.1997**
(21) Numéro de dépôt: 94401149.3
(22) Date de dépôt: 25.05.1994
(51) Int. Cl.: A61K 7/043

(54) **Vernis à ongles aqueux, incoloré ou coloré, contenant un polymère filmogène à l'état dispersé et un composé perfluoroalkyle hydrosoluble**
Ein wässriger, farbiger oder farbloser Nagellack, enthaltend ein filmbildendes Polymer und eine wasserlösliche Perfluoralkyl Verbindung
A coloured or colourless aqueous fingernail varnish containing a dispersed film forming polymer and a water-soluble perfluoroalkyl compound

(30) Priorité: 26.05.1993 FR 9306329
(43) Date de publication de la demande: 07.12.1994
(73) Titulaire: L'OREAL, 75008 Paris (FR)
(72) Inventeur: de la Poterie, Valérie, 77820 Le Chatelet en Brie (FR); Mellul, Myriam, F-94240 l'Hay les Roses (FR)
(74) Mandataire: Stalla-Bourdillon, Bernard

(56) Documents cités:
- EP-A- 0 558 423
- WO-A-88/06434
- WO-A-93/11103
- FR-A- 2 677 982
- GB-A- 1 032 367
- GB-A- 1 074 201

## Description

La présente invention a pour objet une composition cosmétique sous forme d'un vernis à ongles aqueux, coloré ou incolore, contenant un polymère filmogène à l'état dispersé et au moins un composé organique du type perfluoroalkyle hydrosoluble.

A l'heure actuelle, la majeure partie des compositions se présentant sous forme de vernis à ongles sont à base d'un mélange de solvants organiques contenant de la nitrocellulose, une résine aryl-sulfonamide formaldéhyde ou une résine alkyde et un agent plastifiant. De tels vernis, du fait de la présence de solvants organiques, présentent un certain nombre d'inconvénients dans la mesure où ils peuvent endommager les ongles ou la cuticule et provoquer par ailleurs, lors de l'application et du séchage certains dangers à l'égard des utilisatrices.

Depuis quelques années les recherches se sont donc orientées vers la mise au point de vernis à ongles exempts de solvants organiques et en particulier de vernis aqueux.

Si certains résultats ont pu être obtenus à l'aide de tels vernis à ongles aqueux, il s'est avéré que la présence d'une proportion importante d'eau conduisait à un étalement médiocre du vernis sur la surface des ongles se traduisant notamment par la formation de cratères ou d'une rétraction du vernis.

Le problème de l'étalement d'un vernis est en effet primordial pour une application uniforme et un bon recouvrement de la surface de l'ongle.

Ce problème est étroitement lié à la tension superficielle de la composition de vernis à ongles qui doit être la plus basse possible en vue d'obtenir un bon étalement du vernis sur l'ongle.

Il est bien connu de par l'état de la technique que les agents tensio-actifs permettent d'abaisser la tension superficielle des compositions aqueuses. Néanmoins, il s'est avéré que dans le cas des vernis à ongles aqueux, tous les tensio-actifs ne permettaient pas d'apporter cette propriété.

GB-A-1074201 décrit un vernis à ongles aqueux contenant un polymère filmogène et un tensio-actif du type perfluoroalkyle.

Après de nombreuses études, sur un grand nombre d'agents tensio-actifs, on a constaté qu'il était possible d'améliorer dans des conditions particulièrement significatives les propriétés d'étalement des vernis à ongles aqueux en utilisant à titre d'agents tensio-actifs des composés organiques hydrosolubles du type perfluoroalkyle. L'utilisation de tels composés s'est en effet révélée être nettement supérieure à celle d'agents tensio-actifs du type siliconé notamment qui sont connus pour améliorer l'étalement.

L'utilisation dans les compositions de vernis à ongles aqueux d'au moins un composé perfluoroalkyle a pour effet de modifier de façon importante les caractéristiques d'étalement sans pour autant modifier les propriétés intrinsèques du vernis.

La présente invention a donc pour objet à titre de produit industriel nouveau un vernis à ongles aqueux, coloré ou incolore, caractérisé par le fait qu'il contient en plus des ingrédients cosmétiques usuels (a) de 10 à 59 % en poids d'un polymère filmogène présent à l'état dispersé, (b) de 0,01 à 1 % en poids d'un composé perfluoroalkyle dont le radical alkyle a de 3 à 32 atomes de carbone, et (c) de 40 à 90 % en poids d'eau, les proportions de (a), (b) et (c) étant exprimées par rapport au poids total du vernis, ledit composé perfluoroalkyle étant choisi parmi ceux ayant les formules (I), (II) et (III) suivantes :
a)

   (CₙF₂ₙ₊₁)-C₂H₄X (I)

   dans laquelle :
   le radical CₙF₂ₙ₊₁ est linéaire ou ramifié,
   n est compris entre 3 et 16, et
   X représente un radical choisi parmi :
      (i) -CO₂Y
      (ii) -SO₃Y
         Y représentant un atome d'hydrogène, un métal alcalin ou un groupe aminé tel qu'un groupe ammonium.
      (iii) -(OC₂H₄)ₘ-OH
         m étant compris entre 2 et 100, de préférence entre 4 et 40.
      (iv) 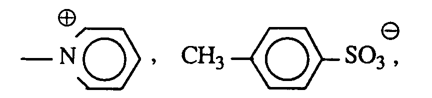
      (v) -SO₂NH(CH₂)₃-N^{⊕}(CH₃)₃I^{⊖}
      (vi) 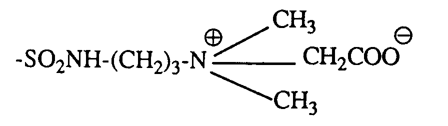
      (vii) 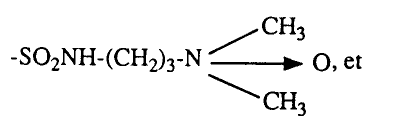 et
      (viii) -SCH₂CH₂R R représentant soit le radical CO₂M, M étant un métal alcalin, en particulier le
         lithium, soit le radical N^{⊕}(CH₃)₃CH₃SO₄^{⊖}
b)

   (CₙF₂ₙ₊₁)-R₁ (II)

   dans laquelle :
   le radical CₙF₂ₙ₊₁ est linéaire ou ramifié,
   n étant compris entre 4 et 16, et
   R₁ est un reste choisi parmi :
      (i) -SO₃^{⊖}NH₄^{⊕}, (ii) -SO₃^{⊖}N^{⊕}(R₃)₄, (iii) -CO₂^{⊖}NH₄^{⊕}, (iv) -CO₂^{⊖}N^{⊕}(R₃)₄,
         R₃ étant un radical alkyle en C₁-C₄,
      (v) -SO₂N(R₃)CH₂-CO₂^{⊖}X^{⊕}
         R₃ étant tel que défini ci-dessus, et
         X est un atome d'hydrogène ou un métal alcalin,
      (vi) SO₂NH(CH₂)ₚN^{⊕}(R₃)₃I^{⊖}
         p étant 1, 2, 3 ou 4, et
         R₃ étant tel que défini ci-dessus, et
      (vii) -SO₂N(R₃)(CH₂CH₂O)-Y
         Y étant un atome d'hydrogène ou un radical alkyle en C₁-C₄, et
         R₃ étant tel que défini ci-dessus, et
c)

   (CₙF₂ₙ₊₁C₂H₄O)ₓP(O)(R)_{y} (III)

   n étant compris entre 3 et 8,
   x et y, différents, représentent 1 ou 2, et
   R représente ONH₄ ou OH.

- Parmi les composés perfluoroalkyles de formule (I), on peut notamment citer ceux correspondant aux formules suivantes :
a) 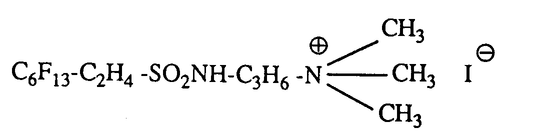 vendu sous la dénomination de "FORARAC 1179" par la Société ATOCHEM;
b) 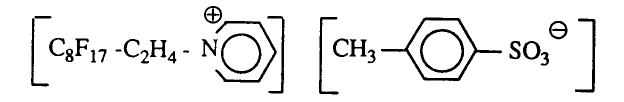 vendu sous la dénomination de "FORAFAC 1098" par la Société ATOCHEM;
c) 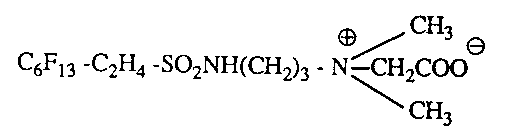 vendu sous la dénomination de "FORAFAC 1157" par la Société ATOCHEM;
d) CₙF₂ₙ₊₁CH₂CH₂O(CH₂CH₂O)ₓH
   n = 3 à 8 et
   x = 2 à 100
   vendu sous les dénominations de "ZONYL-FSN" et "ZONYL FSN.100" par la Société DU PONT ;
e) CₙF₂ₙ₊₁CH₂CH₂SCH₂CH₂CO₂Li
   n = 3 à 8
   vendu sous la dénomination de "ZONYL FSA" par la Société DU PONT ;
f) CₙF₂ₙ₊₁CH₂CH₂SCH₂CH₂N^{⊕}(CH₃)₃CH₃SO₄^{⊖}
   n = 3 à 8
   vendu sous la dénomination de "ZONYL FSC" par la Société DU PONT.

- Parmi les composés perfluoroalkyles de formule (II), on peut notamment citer :
a) CₙF₂ₙ₊₁SO₂N(C₂H₅)CH₂CO₂^{⊖}K^{⊕}
   n = 8
   vendu sous la dénomination de "FLUORAD FC 129" par la Société 3M ;
b) CₙF₂ₙ₊₁SO₂NHC₃H₆N^{⊕}(CH₃)₃I^{⊖}
   n = 8
   vendu sous la dénomination de "FLUORAD FC 135" par la Société 3M ;
c) CₙF₂ₙ₊₁SO₂N(C₂H₅)(CH₂CH₂O)-H
   n = 8
   vendu sous la dénomination de "FLUORAD FC 170C" par la Société 3M ;
d) CₙF₂ₙ₊₁SO₃^{⊖}NH₄^{⊕}
   n = 10
   vendu sous la dénomination de "FLUORAD FC 120" par la Société 3M ;
e) CₙF₂ₙ₊₁SO₂N(C₂H₅)CH₂CO₂^{⊖}NH₄^{⊕}
   n = 8
   vendu sous la dénomination de "FLUORAD FC 143" par la Société 3M.

- Parmi les composés perfluoroalkyles de formule (III), on peut notamment citer :
a) (CₙF₂ₙ₊₁CH₂CH₂O)₁₋₂P(O)(ONH₄)₂₋₁
   n = 3 à 8
   vendu sous la dénomination de "ZONYL FSP, FSE" par la Société DU PONT;
b) (CₙF₂ₙ₊₁CH₂CH₂O)₁₋₂P(O)(OH)₂₋₁
   n = 3 à 8
   vendu sous la dénomination de "ZONYL UR" par la Société DU PONT.

Selon une forme préférée de l'invention, la proportion du composé perfluoroalkyle est de généralement comprise entre 0,05 et 0,2 % en poids par rapport au poids total du vernis à ongles.

Pour la formation des vernis à ongles selon l'invention on utilise de préférence des dispersions aqueuses de polymère fimogène.

Parmi celles-ci on peut notamment mentionner :
- (A) Les dispersions de polyuréthanne de type anionique, cationique ou non ionique, et les dispersions de copolymères de polyuréthanne.
   Parmi ces dispersions on peut notamment citer celles décrites dans : les brevets EP-143.480 et 391.322 et en particulier celles vendues par la Socité ICI sous les dénominations de: "NEOREZ-R974", et de "NEOPAC-E106" et par la Société WITCO Co. sous la dénomination de "WITCOBOND 231".
- (B) Les dispersions de polymères et copolymères acryliques, styréniques, vinyliques et styrène-acryliques.
   Parmi ces dispersions on peut notamment citer celles décrites dans : les demandes de brevets japonais JP 04-103.511, JP-04-103.512, JP-04-103.513, JP-04-103.514, JP-04-103.515, JP-04.103.516, JP-54-52.736, JP-55-70.209 et JP-02-221.214, dans les brevets FR-1.559.020, FR-2.399.238 et FR-2.537.871, dans les brevets US-4.126.144 et 4.116.913, dans le brevet EP-140.325, dans le brevet DE-3.931.237 et dans le brevet CA-1.225.035, et en particulier celles vendues par la Société ICI sous les dénominations de "NEOCRYL-XK53", "NEOCRYL-XK90" et "NEOCRYL-XK62" et par la Société RHONE-POULENC sous la dénomination de "RHODOPASS G5125".

Selon l'invention les vernis à ongles aqueux peuvent contenir divers ingrédients cosmétiques usuels et notamment des pigments organiques et inorganiques.

Parmi les pigments organiques, on peut citer : les D et C Red n° 10, 11, 12 et 13, le D et C Red n° 7, les D et C Red n° 5 et 6, les D et C Red n° 30 et 34, des laques telles que la laque D et C yellow n° 5 et la laque D et C Red n° 2. On peut également citer la guanine.

Parmi les pigments inorganiques, on peut citer : le dioxyde de titane, l'oxychlorure de bismuth, l'oxyde de fer brun et les oxydes de fer rouge.

Selon cette forme de réalisation, les pigments sont généralement présents en une proportion comprise entre 0,05 et 5 % en poids par rapport au poids total du vernis à ongles.

Par ailleurs en vue d'éviter la sédimentation des pigments, certains agents peuvent être employés tels que par exemple des argiles, du silicate de calcium hydraté ou du silicate de magnésium.

Parmi les autres ingrédients des vernis à ongles selon l'invention, on peut citer, les agents conservateurs, les parfums, les plastifiants, les substances filmogènes auxiliaires, les épaississants, les hydratants, les anti-mousse, les cires, les accélérateurs de séchage, les filtres U.V. ainsi qu'en une faible proportion d'autres agents tensio-actifs tels que ceux du type siliconé.

On va maintenant donner à titre d'illustration plusieurs exemples de vernis à ongles aqueux selon l'invention.

Dans les exemples suivants, les lettres "MA" signifient "Matière Active" c'est à dire, dans le cas présent, le polymère filmogène présent en % dans le vernis.

### EXEMPLE 1 : Vernis à ongles coloré

| | |
|---|---|
| - Dispersion de polyuréthanne (à 40 % dans l'eau) vendue par la société ICI sous la dénomination de "NEOREZ-R974" | 70 % (28 % MA) |
| - Pigments | 1,0 % |
| - Conservateurs | 0,05 % |
| - Composé perfluoroalkyle vendu par la Société ATOCHEM sous la dénomination de "FORAFAC 1157" | 0,1 % |
| - Agent épaississant vendu par la société SERVO sous la dénomination de "SERAD FX 1100" | 0,3 % |
| - Eau QSP | 100 % |

Par application de ce vernis sur les ongles, l'étalement est parfaitement uniforme et régulier. Une seule application de vernis est suffisante pour obtenir une couche régulière.

Après séchage, le film obtenu est uniforme et lisse.

### EXEMPLE 2 : Vernis à ongles coloré

| | |
|---|---|
| - Dispersion de polyuréthanne (à 40 % dans l'eau) vendue par la Société ICI sous la dénomination de "NEOREZ R974" | 95 % (38 % MA) |
| - Pigments | 1,5 % |
| - Composé perfluoroalkyle vendu sous la dénomination de "FLUORAD FC143" | 0,2 % |
| - Epaississant du type polyuréthanne vendu par la Société AKZO sous la dénomination de "DAPRAL T210" | 0,3 % |
| - Eau QSP | 100 % |

### EXEMPLE 3 : Vernis à ongles coloré

| | |
|---|---|
| - Dispersion de polyuréthanne (à 40 % dans l'eau) vendue par la société ICI sous la dénomination de "NEOREZ R974" | 75 % (30 % MA) |
| - Pigments | 1,5 % |
| - Composé perfluoroalkyle vendu par la Société DU PONT sous la dénomination de "ZONYL FSP" | 0,2 % |
| - Epaississant du type polyuréthanne vendu par la Société AKZO sous la dénomination de "DAPRAL T210" | 0,4 % |
| - Eau QSP | 100 % |

### EXEMPLE 4 : Vernis à ongles coloré

| | |
|---|---|
| - Dispersion de polyuréthanne (à 40 % dans l'eau) vendue par la société ICI sous la dénomination de "NEOREZ R974" | 70 % (28 % MA) |
| - Pigments | 1,5 % |
| - Composé perfluoroalkyle vendu par la Société ATOCHEM sous la dénomination de "FORAFAC 1157" | 0,2 % |
| - Epaississant du type polyuréthanne vendu par la Société AKZO sous la dénomination de "DAPRAL T210" | 0,3 % |
| - Eau QSP | 100 % |

### EXEMPLE 5 : Vernis à ongles coloré

| | |
|---|---|
| - Dispersion de polyuréthanne (à 40 % dans l'eau) vendue par la société ICI sous la dénomination de "NEOREZ R974" | 50 % (20 % MA) |
| - Pigments | 1,5 % |
| - Composé perfluoroalkyle vendu par la Société 3M sous la dénomination de "FLUORAD FC143" | 0,1 % |
| - Epaississant du type polyuréthanne vendu par la Société AKZO sous la dénomination de "DAPRAL T210" | 0,5 % |
| - Eau QSP | 100 % |

### EXEMPLE 6 : Vernis à ongles coloré

| | |
|---|---|
| - Dispersion de polyuréthanne (à 40 % dans l'eau) vendue par la société ICI sous la dénomination de "NEOREZ R974" | 95 % (38 % MA) |
| - Pigments | 1,5 % |
| - Composé perfluoroalkyle vendu par la Société ATOCHEM sous la dénomination de "FORAFAC 1157" | 0,1 % |
| - Epaississant du type polyuréthanne vendu par la Société AKZO sous la dénomination de "DAPRAL T212" | 0,3 % |
| - Eau QSP | 100 % |

### EXEMPLE 7 : Vernis à ongles coloré

| | |
|---|---|
| - Dispersion de polyuréthanne (à 30 % dans l'eau) vendue par la société WITCO sous la dénomination de "WITCOBOND 231" | 90 % (27 % MA) |
| - Pigments | 1,5 % |
| - Composé perfluoroalkyle vendu par la Société ATOCHEM sous la dénomination de "FLUORAD FC143" | 0,2 % |
| - Epaississant du type polyuréthanne vendu par la Société AKZO sous la dénomination de "DAPRAL T210" | 0,3 % |
| - Eau QSP | 100 % |

### EXEMPLE 8 : Vernis à ongles coloré

| | |
|---|---|
| - Dispersion polyacrylique (à 45 % dans l'eau) vendue par la Société ICI sous la dénomination de "NEOCRYL XK90" | 60 % (27 % MA) |
| - Pigments | 1,5 % |
| - Composé perfluoroalkyle vendu par la Société ATOCHEM sous la dénomination de "FLUORAD FC143" | 0,2 % |
| - Epaississant du type polyuréthanne vendu par la Société AKZO sous la dénomination de "DAPRAL T210" | 0,3 % |
| - Eau QSP | 100 % |

### EXEMPLES COMPARATIFS DE VERNIS à ONGLES

### EXEMPLE A :

| | |
|---|---|
| - Dispersion de polyuréthanne (à 40 % dans l'eau) vendue par la société ICI sous la dénomination de "NEOREZ R974" | 95 % (38 % MA) |
| - Pigments | 1,5 % |
| - Epaississant du type polyuréthanne vendu par la Société AKZO sous la dénomination de "DAPRAL T210" | 0,3 % |
| - Eau QSP | 100 % |

### EXEMPLE B :

| | |
|---|---|
| - Dispersion de polyuréthanne (à 40 % dans l'eau) vendue par la société ICI sous la dénomination de "NEOREZ R974" | 95 % (38 % MA) |
| - Pigments | 1,5 % |
| - Tensio-actif siliconé vendu par la Société SHIN ETSU sous la dénomination de "KF 355A" | 0,2 % |
| - Epaississant du type polyuréthanne vendu par la Société AKZO sous la dénomination de "DAPRAL T210" | 0,3 % |
| - Eau QSP | 100 % |

### EXEMPLE C :

| | |
|---|---|
| - Dispersion de polyuréthanne (à 40 % dans l'eau) vendue par la société ICI sous la dénomination de "NEOREZ R974" | 95 % (38 % MA) |
| - Pigments | 1,5 % |
| - Tensio-actif siliconé vendu par la Société SHIN ETSU sous la dénomination de "KF 355A" | 0,1 % |
| - Epaississant du type polyuréthanne vendu par la Société AKZO sous la dénomination de "DAPRAL T210" | 0,3 % |
| - Eau QSP | 100 % |

Les vernis des exemples 2 à 6 s'étalent de façon régulière et uniforme. Une seule application de vernis suffit pour obtenir une couche régulière et un film lisse.

Les vernis des exemples comparatifs A à C qui ne contiennent pas de composé perfluoroalkyle ou qui contiennent à la place un composé siliconé, ne s'étalent pas de manière uniforme et régulière. Le film n'est pas homogène et il est nécessaire d'appliquer plusieurs fois le vernis pour obtenir une couche régulière.

## Revendications

1. Vernis à ongles aqueux, coloré ou incolore, caractérisé par le fait qu'il contient en plus des ingrédients usuels (a) de 10 à 59 % en poids d'un polymère filmogène présent à l'état dispersé (b) de 0,01 à 1 % en poids d'un composé perfluoroalkyle hydrosoluble dont le radical alkyle a de 3 à 32 atomes de carbone et (c) de 40 à 90 % en poids d'eau, les proportions de (a), (b) et (c) étant exprimées par rapport au poids total du vernis, ledit composé perfluoroalkyle répondant à l'une des formules suivantes :
a)
(CₙF₂ₙ₊₁)-C₂H₄X (I)
dans laquelle :
le radical CₙF₂ₙ₊₁ est linéaire ou ramifié,
n est compris entre 4 et 16, et
X représente un radical choisi parmi :
(i) -CO₂Y
(ii) -SO₃Y
Y représentant un atome d'hydrogène, un métal alcalin ou un groupe aminé tel qu'un groupe ammonium.
(iii) -(OC₂H₄)ₘ-OH
m étant compris entre 2 et 100, de préférence entre 4 et 40.
(iv) 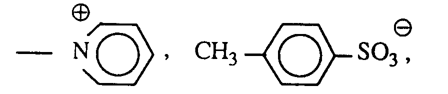
(v) -SO₂NH(CH₂)₃-N^{⊕}(CH₃)₃I^{⊖},
(vi) 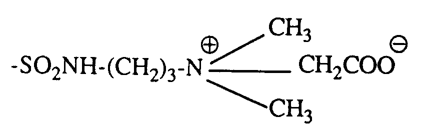
(vii) 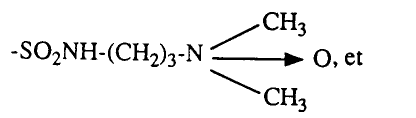 et
(viii) -SCH₂CH₂R
R représentant soit le radical CO₂M, M étant un métal alcalin, en particulier le lithium, soit le radical N^{⊕}(CH₃)₃CH₃SO₄^{⊖}.
b)
(CₙF₂ₙ₊₁)-R₁ (II)
dans laquelle :
le radical CₙF₂ₙ₊₁ est linéaire ou ramifié,
n étant compris entre 4 et 16, et
R₁ est un reste choisi parmi :
(i) -SO₃^{⊖}NH₄^{⊕}, (ii) -SO₃^{⊖}N^{⊕}(R₃)₄, (iii) -CO₂^{⊖}NH₄^{⊕}, (iv) -CO₂^{⊖}N^{⊕}(R₃)₄,
R₃ étant un radical alkyle en C₁-C₄,
(v) -SO₂N(R₃)CH₂-CO₂^{⊖}X^{⊕}
R₃ étant tel que défini ci-dessus, et
X est un atome d'hydrogène ou un métal alcalin,
(vi) -SO₂NH(CH₂)ₚN^{⊕}(R₃)₃I^{⊖}
p étant 1, 2, 3 ou 4, et
R₃ étant tel que défini ci-dessus, et
(vii) -SO₂N(R₃)(CH₂CH₂O)-Y
Y étant un atome d'hydrogène ou un radical alkyle en C₁-C₄, et
R₃ étant tel que défini ci-dessus, et
c)
(CₙF₂ₙ₊₁C₂H₄O)ₓP(O)(R)_{y} (III)
n étant compris entre 3 et 8,
x et y différents, représentent 1 ou 2, et
R représente ONH₄ ou OH.

2. Vernis à ongles selon la revendication 1, caractérisé par le fait que le composé perfluoroalkyle est présent en une proportion comprise entre 0,05 et 0,2 % en poids par rapport au poids total du vernis à ongles.

3. Vernis à ongles selon le revendication 1 caractérisé par le fait que le polymère filmogène est choisi parmi les polyuréthannes de type anionique, cationique ou non ionique, les copolymères de polyuréthanne, et les polymères et copolymères acryliques, styréniques, vinyliques, et styrène-acryliques.

4. Vernis à ongles selon l'une quelconque des revendications précédentes caractérisé par le fait qu'il contient au moins un pigment organique ou inorganique en une proportion comprise entre 0,05 et 5 % en poids par rapport au poids total du vernis.

5. Vernis à ongles selon l'une quelconque des revendications précédentes caractérisé par le fait qu'il contient en outre au moins un agent permettant d'éviter la sédimentation, un agent conservateur, un parfum, un plastifiant, une substance filmogène auxilliaire, un épaississant, un hydratant, un anti-mousse, une cire, un accélérateur de séchage, un filtre U.V. et un agent tensio-actif du type siliconé ou un mélange de ceux-ci.

## Claims

1. Coloured or colourless, aqueous nail varnish characterized in that in addition to the usual ingredients it contains (a) from 10 to 59 % by weight of a film-forming polymer present in the dispersed state, (b) from 0.01 to 1 % by weight of a water-soluble perfluoroalkyl compound in which the alkyl radical contains from 3 to 32 carbon atoms, and (c) from 40 to 90 % by weight of water, the proportions of (a), (b) and (c) being expressed in relation to the total weight of the varnish, the said perfluoroalkyl compound corresponding to one of the following formulae:
a)
(CₙF₂ₙ₊₁)-C₂H₄X (I)
in which:
the radical CₙF₂ₙ₊₁ is linear or branched,
n is between 4 and 16, and
X denotes a radical chosen from:
(i) -CO₂Y
(ii) -SO₃Y
Y denoting a hydrogen atom, an alkali metal or an amino group such as an ammonium group,
(iii) -(OC₂H₄)ₘ-OH
m being between 2 and 100, preferably between 4 and 40,
(iv) 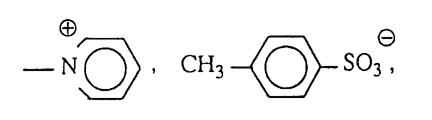
(v) -SO₂NH(CH₂)₃-N^{⊕}(CH₃)₃I^{⊖}
(vi) 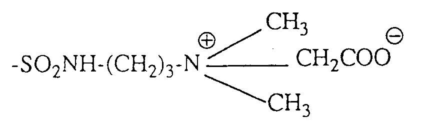
(vii) 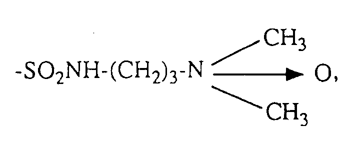 and
(viii) -SCH₂CH₂R
R denoting either the radical CO₂M, M being an alkali metal, in particular lithium, or the radical N^{⊕}(CH₃)₃ CH₃SO₄^{⊖},
b)
(CₙF₂ₙ₊₁)-R₁ (II)
in which:
the radical CₙF₂ₙ₊₁ is linear or branched,
n being between 4 and 16, and
R₁ is a residue chosen from:
(i) -SO₃^{⊖}NH₄^{⊕}, (ii) -SO₃^{⊖}N^{⊕}(R₃)₄, (iii) -CO₂^{⊖}NH₄^{⊕}, (iv) -CO₂^{⊖}N^{⊕}(R₃)₄,
R₃ being a C₁-C₄ alkyl radical,
(v) -SO₂N(R₃)CH₂-CO₂^{⊖}X^{⊕}
R₃ being as defined above, and
X is a hydrogen atom or an alkali metal,
(vi) -SO₂NH(CH₂)ₚN^{⊕}(R₃)₃I^{⊖}
p being 1, 2, 3 or 4, and
R₃ being as defined above, and
(vii) -SO₂N(R₃)(CH₂CH₂O)-Y
Y being a hydrogen atom or C₁-C₄ alkyl radical, and
R₃ being as defined above, and
c)
(CₙF₂ₙ₊₁C₂H₄O)ₓP(O)(R)_{y} (III)
n being between 3 and 8,
x and y, which are different, denote 1 or 2, and
R denotes ONH₄ or OH.

2. Nail varnish according to Claim 1, characterized in that the perfluoroalkyl compound is present in a proportion of between 0.05 and 0.2 % by weight relative to the total weight of the nail varnish.

3. Nail varnish according to Claim 1, characterized in that the film-forming polymer is chosen from polyurethanes of anionic, cationic or nonionic type, polyurethane copolymers and acrylic, styrene-based, vinyl and styrene-acrylic polymers and copolymers.

4. Nail varnish according to any one of the preceding claims, characterized in that it contains at least one organic or inorganic pigment in a proportion of between 0.05 and 5 % by weight relative to the total weight of the varnish.

5. Nail varnish according to any one of the preceding claims, characterized in that it additionally contains at least one agent which makes it possible to prevent settling, a preserving agent, a perfume, a plasticizer, an auxiliary film-forming substance, a thickener, a hydrating agent, an antifoam, a wax, a drying accelerator, a UV filter and a surface-active agent of the silicone type, or a mixture thereof.

## Patentansprüche

1. Wäßriger farbiger oder farbloser Nagellack, dadurch gekennzeichnet, daß er zusätzlich zu den üblichen Bestandteilen
(a) 10 bis 59 Gew.-% eines filmbildenden Polymers in dispergiertem Zustand,
(b) 0,01 bis 1 Gew.-% einer wasserlöslichen Perfluoralkylverbindung, dessen Alkylrest 3 bis 32 Kohlenstoffatome aufweist, und
(c) 40 bis 90 Gew.-% Wasser enthält,
wobei die jeweiligen Anteile an (a), (b) und (c) in bezug auf das jeweilige Gesamtgewicht des Nagellacks ausgedrückt sind und die Perfluoralkylverbindung einer der folgenden Formeln entspricht:
a)
(CₙF₂ₙ₊₁)-C₂H₄X (I)
worin
der Rest CₙF₂ₙ₊₁ gerad- oder verzweigtkettig ist,
n zwischen 4 und 16 beträgt, und
X einen Rest darstellt, der unter den folgenden ausgewählt ist:
(i) -CO₂Y
(ii) -SO₃Y
wobei Y ein Wasserstoffatom, ein Alkalimetall oder eine Aminogruppe wie zum Beispiel eine Ammoniumgruppe bedeutet;
(iii) -(OC₂H₄)ₘ-OH
wobei m zwischen 2 und 100, vorzugsweise zwischen 4 und 40 beträgt;
(iv) 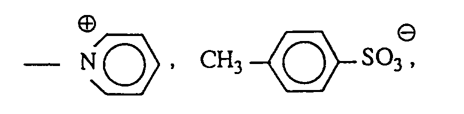
(v) -SO₂NH(CH₂)₃-N^{⊕}(CH₃)₃I^{⊖},
(vi) 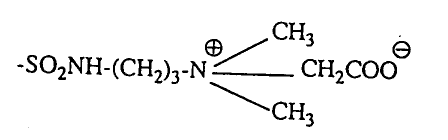
(vii) 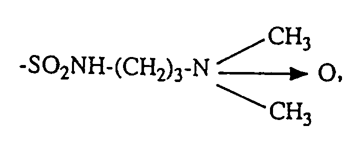 sowie
(viii) -SCH₂CH₂R
wobei R entweder den Rest -CO₂M bedeutet, wobei M ein Alkalimetall, insbesondere Lithium bedeutet, oder den Rest N^{⊕}(CH₃)₃CH₃SO₄^{⊖} darstellt;
b)
(CₙF₂ₙ₊₁)-R₁ (II)
worin
der Rest CₙF₂ₙ₊₁ gerad- oder verzweigtkettig ist,
wobei n zwischen 4 und 16 beträgt, und
R₁ einen Rest darstellt, der unter den folgenden ausgewählt ist:
(i) -SO₃^{⊖}NH₄^{⊕},
(ii) -SO₃^{⊖}N^{⊕}(R₃)₄,
(iii) -CO₂^{⊖}NH₄^{⊕},
(iv) -CO₂^{⊖}N^{⊕}(R₃)₄,
wobei
R₃ einen C₁-C₄-Alkylrest bedeutet,
(v) -SO₂N(R₃)CH₂-CO₂^{⊖}X^{⊕}
wobei
R₃ die vorstehende Bedeutung aufweist, und
X ein Wasserstoffatom oder eine Alkalimetall bedeutet,
(vi) -SO₂NH(CH₂)ₚN^{⊕}(R₃)₃I^{⊖}
wobei
p 1, 2, 3 oder 4 bedeutet, und
R₃ die vorstehende Bedeutung aufweist, sowie
(vii) -SO₂N(R₃)(CH₂CH₂O)-Y
wobei
Y ein Wasserstoffatom oder einen C₁-C₄-Rest darstellt, und
R₃ die vorstehende Bedeutung aufweist, und
c)
(CₙF₂ₙ₊₁C₂H₄O)ₓP(O)(R)_{y} (III)
wobei
n zwischen 3 und 8 beträgt,
x und y voneinander verschieden sind sowie 1 oder 2 bedeuten, und
R die Gruppe ONH₄ oder OH bedeutet.

2. Nagellack nach Anspruch 1, dadurch gekennzeichnet,daß die Perfluoralkylverbindung in einem Mengenverhältnis zwischen 0,05 Gew.-% und 0,2 Gew.-% in bezug auf das Gesamtgewicht des Nagellacks vorhanden ist.

3. Nagellack nach Anspruch 1, dadurch gekennzeichnet, daß das filmbildende Polymer unter Polyurethanen vom anionischen, kationischen oder nichtionischen Typ, aus Polyurethancopolymeren, sowie den Acrylsäure-, Styrol-, Vinyl- und Styrol/Acrylsäure-Polymeren und -Copolymeren ausgewählt ist.

4. Nagellack nach einem der vorstehenden Ansprüche, dadurch gekennzeichnet, daß er mindestens ein organisches oder anorganisches Pigment in einem Mengenverhältnis zwischen 0,05 Gew-% und 5 Gew.-% in bezug auf das Gesamtgewicht des Nagellacks enthält.

5. Nagellack nach einem der vorstehenden Ansprüche, dadurch gekennzeichnet, daß er zusätzlich noch mindestens ein Mittel zum Vermeiden des Absetzens, ein Konservierungsmittel, einen Duftstoff, Weichmacher, eine Hilfsfilmbildnersubstanz, ein Verdickungsmittel, Hydratisierungsmittel, Antischaummittel, Wachs, einen Trocknungsbeschleuniger, eine UV-Filtersubstanz sowie ein Tensid vom Silikontyp oder eine Mischung daraus enthält.
